# EUROPEAN PATENT APPLICATION

(11) **EP 4 145 327 A1**
(43) Date of publication of application: **08.03.2023**
(21) Application number: 21796302.4
(22) Date of filing: 09.04.2021
(51) Int. Cl.: G06F 30/10, G06F 30/27

(54) **SYSTEM FOR ESTIMATING CHARACTERISTIC VALUE OF MATERIAL**

(30) Priority: 28.04.2020 JP 2020079791
(71) Applicant: Hitachi, Ltd., Tokyo 100-8280 (JP)
(72) Inventor: KANAZAWA Takuya, Tokyo 100-8280 (JP); ASAHARA Akinori, Tokyo 100-8280 (JP); MORITA Hidekazu, Tokyo 100-8280 (JP)
(74) Representative: MERH-IP Matias Erny Reichl Hoffmann Patentanwälte PartG mbB
(86) International application number: PCT/JP2021/015043
(87) International publication number: WO 2021/220775

(57) **Abstract**

A simulation estimation model estimates a feature value of a simulation result of a material from a descriptor of the material. A material feature value estimation model estimates a feature value of the material from the estimation result of the simulation estimation model and the descriptor of the material. One or more processors input a descriptor of a first material into the simulation estimation model to acquire a first simulation estimation result of the feature value of the first material. The one or more processors input the first simulation estimation result and the descriptor of the first material into the material feature value estimation model to acquire the feature estimation value of the first material.

## Description

### Incorporation by Reference

This application claims priority to Japanese Patent Application No. 2020-079791 filed on April 28, 2020, the content of which is incorporated herein by reference.

### Technical Field

The present invention relates to a system for estimating a feature value of material.

### Background Art

As a method different from an evaluation method of material feature by tests, material feature evaluation by numerical simulation has been performed. In the numerical simulation of a material, a simulator is configured on the basis of physical laws, and a material descriptor is input to the numerical simulator, thereby obtaining a material feature value as a simulation result. Material informatics estimates a feature value of a material using a machine learning model that considers only a response relationship between a feature of the material and the feature value, and selects a target for test and numerical simulation. This makes it possible to optimize the number of times.

In such a situation, NPL 1 discloses a technique of performing material feature estimation by a machine learning model by using a numerical simulation result of a material for learning data of machine learning. In addition, PTL 1 discloses a technique of improving generalization performance of a machine learning model by creating a 3D model from image data, performing physical simulation of the model, and generating a large amount of new learning image data.

### Citation List

### Patent Literature

PTL 1: JP 2017-182129 A

### Non Patent Literature

NPL 1: G. R. Schleder et al., "From DFT to machine learning: recent approaches to materials science-a review", J. Phys.: Mater. 2 (2019) 032001

### Summary of Invention

### Technical Problem

A numerical simulation of a material, particularly a numerical simulation by first-principle calculation such as density functional method, can give a good approximate value of a material feature value that is a target of estimation. However, the calculation cost of the numerical simulation is extremely high, and the number of material types for which the numerical simulation can be executed is limited. Therefore, there is a demand for a technique capable of replacing the numerical simulation with a machine learning model and estimating the material feature value with high accuracy with less calculation cost.

### Solution to Problem

One aspect of the present invention is a system that estimates a feature value of a material, the system including one or more processors and one or more storage devices. The one or more storage devices store a material feature estimation model. The material feature estimation model includes a simulation estimation model that estimates a feature value of a simulation result of a material from a descriptor of the material, and a material feature value estimation model that estimates a feature value of the material from an estimation result of the simulation estimation model and a descriptor of the material. The one or more processors inputs a descriptor of a first material into the simulation estimation model to acquire a first simulation estimation result of a feature value of the first material, and inputs the first simulation estimation result and a descriptor of the first material into the material feature value estimation model to acquire a feature estimation value of the first material.

### Advantageous Effects of Invention

According to one aspect of the present invention, a material feature value can be highly accurately and efficiently estimated by a machine learning model.

### Brief Description of Drawings

[FIG. 1] FIG. 1 schematically illustrates a material feature estimation model that can substitute for a numerical simulator according to an example of the present description.
[FIG. 2] FIG. 2 schematically illustrates a logic configuration example of a material feature estimation device according to an example of the present description.
[FIG. 3] FIG. 3 illustrates a hardware configuration example of a material feature estimation device.
[FIG. 4] FIG. 4 illustrates a configuration example of a tested material database.
[FIG. 5] FIG. 5 illustrates a configuration example of an untested material database.
[FIG. 6] FIG. 6 illustrates a configuration example of a descriptor list output by a descriptor calculation unit.
[FIG. 7] FIG. 7 illustrates a flowchart of an example of overall processing of the material feature estimation device.
[FIG. 8] FIG. 8 schematically illustrates distribution of a material in a two-dimensional space.
[FIG. 9] FIG. 9 illustrates a flowchart of details of learning of a simulation estimation model.
[FIG. 10] FIG. 10 illustrates a flowchart of details of learning of a material feature value estimation model.
[FIG. 11] FIG. 11 illustrates an image example of a material feature estimation result displayed on a monitor by a material feature estimation result display unit.

### Description of Embodiments

In the following, when it is necessary for convenience, the description will be divided into a plurality of sections or examples, but unless otherwise specified, they are not unrelated to one another, and they are in a relationship where one is a modification, detail, supplementary explanation, and the like of some or all of the others. In the following, when referring to the number of elements and the like (including number of items, numerical value, amount, range, and the like), the number is not limited to a specific number unless otherwise stated or unless clearly limited to the specific number in principle, and the number may be equal to greater than or equal to or less than the specific number.

The present system may be a physical computer system (one or more physical computers) or a system constructed on a calculation resource group (a plurality of calculation resources) such as a cloud infrastructure. The computer system or the calculation resource group includes one or more interface devices (for example, including a communication device and an input/output device), one or more storage devices (for example, including a memory (main storage) and an auxiliary storage device), and one or more processors.

In a case where the function is implemented by executing a program by a processor, determined processing is appropriately performed using the storage device and/or the interface device, and thus, the function may be at least a part of the processor. The processing described with the function as the subject may be processing performed by a processor or a system including the processor. The program may be installed from a program source. The program source may be, for example, a program distribution computer or a computer-readable storage medium (for example, a computer-readable non-transitory storage medium). The description of each function is an example, and a plurality of functions may be put together into one function or one function may be divided into a plurality of functions.

### [Outline]

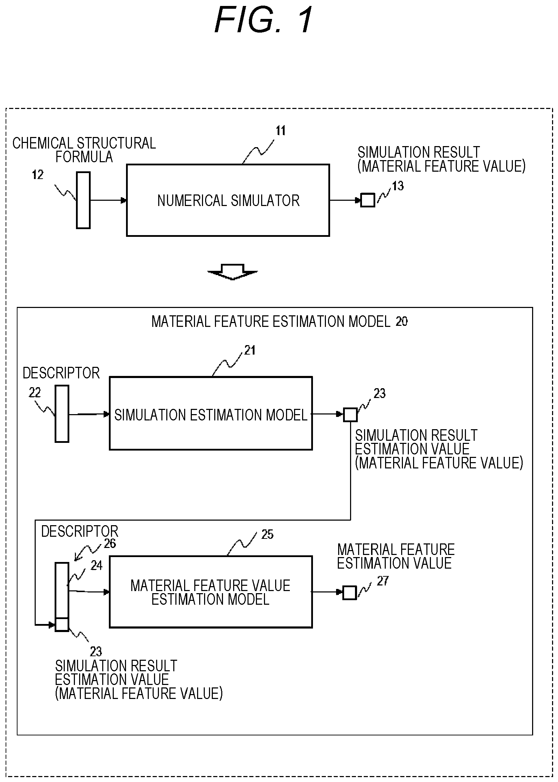

Hereinafter, a technique capable of efficiently and highly accurately estimating a material feature value will be disclosed. Examples of the present description enable a numerical simulator of a material feature to be replaced by a machine learning model (material feature estimation model). FIG. 1 schematically illustrates a material feature estimation model 20 that can substitute for a numerical simulator 11 in an example of the present description.

The numerical simulator 11 outputs a simulation result 13 of a predetermined feature value of a material from a chemical structural formula 12 of the material that has been input. In the example of FIG. 1, the numerical simulator 11 receives a chemical structural formula as input and outputs one type of material feature value, but in another example, the numerical simulator 11 may receive a descriptor of a chemical structural formula as input and may output a plurality of types of material feature values.

The material feature estimation model 20 includes a simulation estimation model 21 that estimates a simulation result of the numerical simulator 11 and a material feature value estimation model 25. The simulation estimation model 21 receives a descriptor 22 (vector) of a material as input, and estimates a simulation result (material feature value) of the numerical simulator 11. The descriptor is a vector representing a feature of a material in a multivariate manner.

The descriptor includes a plurality of elements (feature), and represents a feature corresponding to each element, for example, a molecular weight or an element mixing ratio. In the example of FIG. 1, the simulation estimation model 21 outputs one type of material feature value, but may output a plurality of types of material feature values included in the simulation result of the numerical simulator 11. The simulation estimation model 21 is optimized (trained) based on an error between the simulation result 13 of the numerical simulator 11 and an estimation result 23 of the simulation estimation model 21.

The material feature value estimation model 25 estimates one or a plurality of types of material feature values that are identical to the material feature value estimated by the simulation estimation model 21. In the example of FIG. 1, the material feature value estimation model 25 estimates one specific type of material feature value.

The material feature value estimation model 25 receives, as input, a vector 26 in which a descriptor 24 of the material and the simulation result estimation value 23 of the material feature estimation model 20 are combined. The descriptor 24 may be identical to or different from the descriptor 22 input to the simulation estimation model 21. The vector 26 is a descriptor in which the descriptor 22 of the material is extended. The material feature value estimation model 25 estimates a predetermined material feature value from an extension descriptor 26, and outputs its material feature estimation value 27. The material feature estimation value 27 is an estimation value of the material feature by the material feature estimation model 20.

As described above, the material feature value estimation model 25 estimates the feature value of the material on the basis of the estimation result of the simulation estimation model 21 that estimates the simulation result of the numerical simulator 11 and the descriptor of the material. Due to this, the material feature value can be estimated with high accuracy by the machine learning model that can perform arithmetic operation more efficiently than the simulator.

Note that regression algorithms used by the simulation estimation model 21 and the material feature value estimation model 25 are discretionary, and these algorithms may be identical or different. For example, a discretionary algorithm can be selected from various regression algorithms including random forest, support vector machine, Gaussian process regression, and neural network. The material feature estimation model 20 is applicable to any of an organic-inorganic compound and an inorganic compound. The descriptor can be generated from a chemical formula, that is, any of a structural formula and a composition formula. Hereinafter, a more specific configuration of the example of the present description will be described.

### [Example 1]

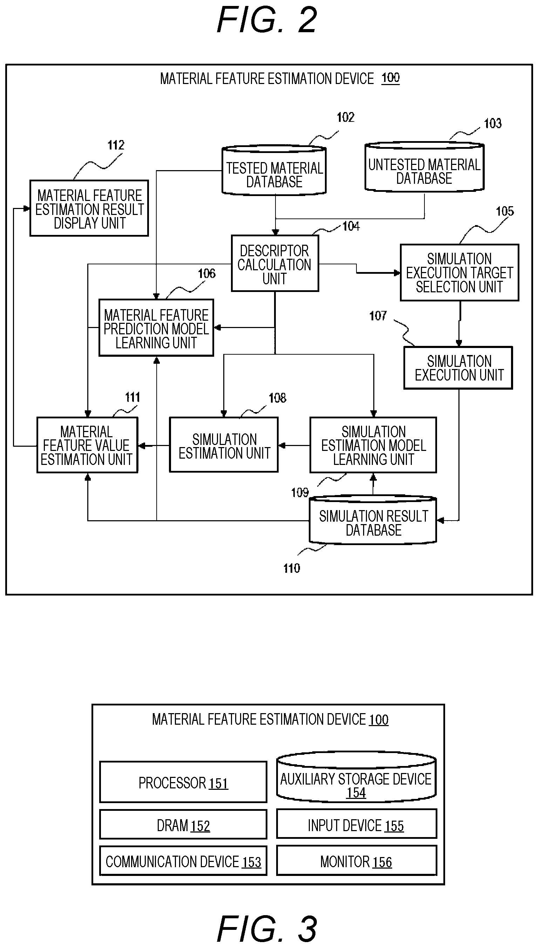

FIG. 2 schematically illustrates a logic configuration example of the material feature estimation device according to the example of the present description. A material feature estimation device 100 stores a tested material database 102, an untested material database 103, and a simulation result database 110.

The material feature estimation device 100 stores a descriptor calculation unit 104, a simulation execution target selection unit 105, a material feature value estimation model learning unit 106, a simulation execution unit 107, a simulation estimation unit 108, a simulation estimation model learning unit 109, a material feature value estimation unit 111, and a material feature estimation result display unit 112. These are programs, and one or more processors of the material feature estimation device 100 can operate as corresponding functional units by executing these programs. Note that a discretionary function of the material feature estimation device 100 can be implemented in a discretionary program.

The descriptor calculation unit 104 generates a descriptor from a chemical formula by a predetermined method. The descriptor represents a feature of the material indicated by the chemical formula. The descriptor is represented by a vector including a plurality of elements (feature). A feature corresponding to each element, for example, a molecular weight or an element mixing ratio is represented. Hereinafter, the organic compound material represented by the chemical structural formula will be described as an example of the estimation target material. Examples of the present description are also applicable to an inorganic compound material represented by a composition formula, for example.

The simulation execution target selection unit 105 selects a material for which simulation is to be executed by the numerical simulator 11 in order to generate learning data for learning (training) the material feature estimation model 20. The simulation execution unit 107 executes simulation by the numerical simulator 11.

The simulation estimation model learning unit 109 performs learning (training) of the simulation estimation model 21 that estimates a simulation result. The simulation estimation unit 108 calculates a simulation result estimation value of the material feature by the learned simulation estimation model 21.

The material feature value estimation model learning unit 106 performs learning (training) of the material feature value estimation model 25 that estimates a material feature value. The material feature value estimation unit 111 calculates an estimation value of the material feature value by the learned material feature value estimation model 25. The material feature estimation result display unit 112 presents the user a material feature estimation result by the material feature value estimation unit 111.

The tested material database 102 stores test results of predetermined material feature values of various materials. The untested material database 103 stores data of materials for which tests on material feature values have not been executed. The simulation result database 110 stores a simulation result by the numerical simulator 11.

FIG. 3 illustrates a hardware configuration example of the material feature estimation device 100. The material feature estimation device 100 has a computer configuration and includes a processor 151 having calculation performance and a DRAM 152 that gives a volatile temporary storage area for storing programs to be executed by the processor 151 and data. The material feature estimation device 100 further includes a communication device 153 that performs data communication with another device, and an auxiliary storage device 154 that gives a permanent information storage area using a hard disk drive (HDD), a flash memory, and the like.

For example, the auxiliary storage device 154 stores programs such as the descriptor calculation unit 104, the simulation execution target selection unit 105, the material feature value estimation model learning unit 106, the simulation execution unit 107, the simulation estimation unit 108, the simulation estimation model learning unit 109, the material feature value estimation unit 111, and the material feature estimation result display unit 112.

The auxiliary storage device 154 further stores various data such as the tested material database 102, the untested material database 103, and the simulation result database 110. The program to be executed by the processor 151 and processing target data are loaded from the auxiliary storage device 154 to the DRAM 152.

The material feature estimation device 100 includes an input device 155 that receives operation from the user, and a monitor 156 (example of an output device) that presents the user an output result in each process. Note that the function of the material feature estimation device 100 may be separately implemented in a plurality of devices. Thus, the material feature estimation device 100 includes one or more storage devices and one or more processors.

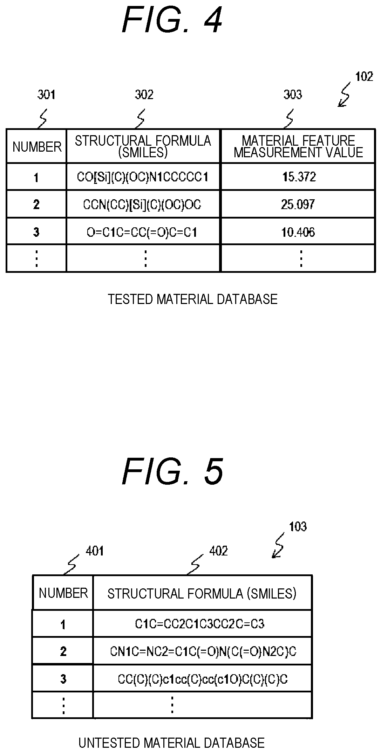

FIG. 4 illustrates a configuration example of the tested material database 102. The tested material database 102 associates a material with a test result of a feature value of the material. Specifically, the tested material database 102 includes a number column 301, a structural formula (SMILES) column 302, and a material feature measurement value column 303.

The number column 301 identifies each record in the tested material database 102. The structural formula (SMILES) column 302 indicates the chemical structural formula of the material. In the example of FIG. 4, the chemical structural formula is expressed according to simplified molecular input line entry system (SMILES) notation. It is possible to use a discretionary expression format of a chemical structural formula that can generate a descriptor. The material feature measurement value column 303 indicates a test result of a predetermined feature value of each chemical structural formula.

FIG. 5 illustrates a configuration example of the untested material database 103. The untested material database 103 stores a chemical structural formula of a material for which a test of the material feature value has not been conducted. The feature value of the material selected from the untested material database 103 is estimated by the material feature estimation model 20.

In the example shown in FIG. 5, the untested material database 103 includes a number column 401 and a structural formula (SMILES) column 402. The number column 401 identifies each record in the untested material database 103. The structural formula (SMILES) column 402 indicates SMILES expression of a chemical structural formula of a material.

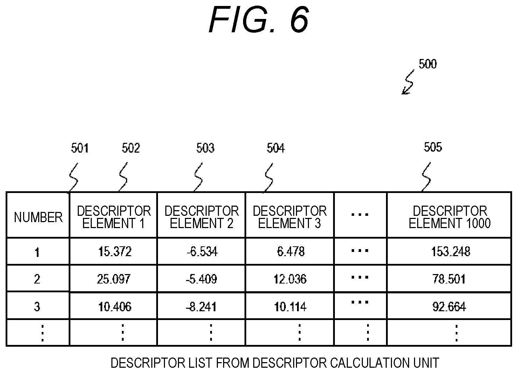

FIG. 6 illustrates a configuration example of a descriptor list 500 output by the descriptor calculation unit 104. The descriptor calculation unit 104 generates a descriptor from the chemical structural formula of the SMILES expression acquired from the tested material database 102 or the untested material database 103, and generates the descriptor list 500.

The descriptor list 500 includes a number column 501 and a column of each descriptor element. In the example of FIG. 6, the descriptor includes 1000 description elements, and columns of four descriptor elements are indicated by reference numerals 502 to 505 as an example. The value of the number column 501 corresponds to the value of the number column in the database from which the chemical structural formula for generating the descriptor list has been acquired.

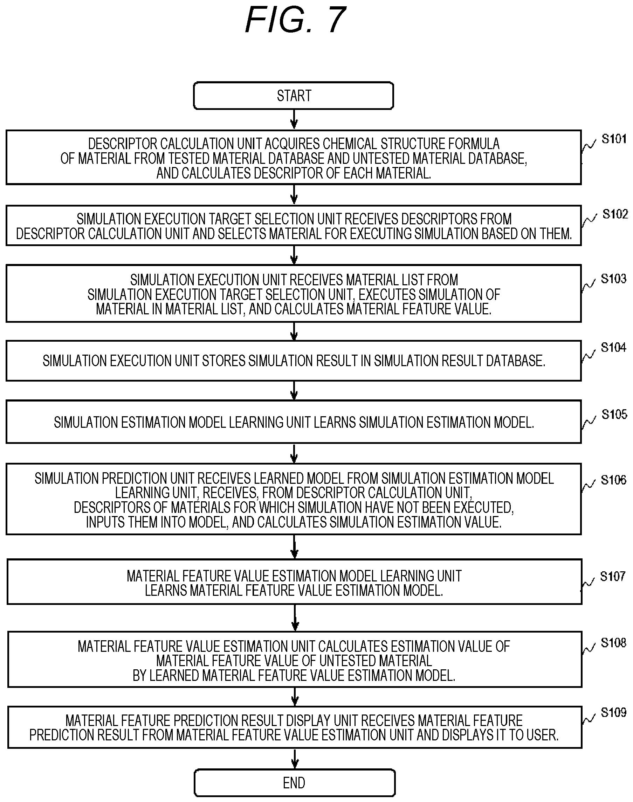

FIG. 7 illustrates a flowchart of an example of overall processing of the material feature estimation device 100. In step S101, the descriptor calculation unit 104 acquires a chemical structural formula of a material from the tested material database 102 and the untested material database 103, and calculates a descriptor of each material. The descriptor calculation unit 104 generates a descriptor list of each of the tested material database 102 and the untested material database 103.

In step S102, the simulation execution target selection unit 105 receives the descriptor of the material of each of the two databases 102 and 103 from the descriptor calculation unit 104, and selects the materials for which simulation is executed on the basis of the descriptors. The simulation result is used for learning of the material feature estimation model 20.

The numerical simulation requires many calculation resources. From the viewpoint of efficient and effective learning of the material feature estimation model 20, it is important to select a material for which simulation is to be executed by the numerical simulator 11.

From the viewpoint of learning of the simulation estimation model 21, it is possible to improve generality of the simulation estimation model 21 by preparing simulation results of various types of qualitatively different materials (request 1). For the purpose of learning of the material feature value estimation model 25, it is necessary to execute numerical simulation on a tested material (request 2) .

The simulation execution target selection unit 105 determines the priority order of numerical simulation candidates so as to satisfy the requests 1 and 2, and selects a higher-order material as a simulation target.

From the viewpoint of the request 1, the simulation execution target selection unit 105 determines the simulation execution target on the basis of the similarity between materials. The similarity between materials can be calculated from a distance between, for example, descriptors or vectors obtained from descriptors.

For example, the simulation execution target selection unit 105 reduces the dimension of the descriptor of a candidate material, and analyzes the distribution of the materials in a low-dimensional space. For dimension reduction, for example, a dimension reduction algorithm such as t-distributed stochastic neighbor embedding (t-SNE) can be used. A predetermined element of the descriptor may be extracted to constitute a low-dimensional space. The subsequent calculation amount is reduced by the dimension reduction.

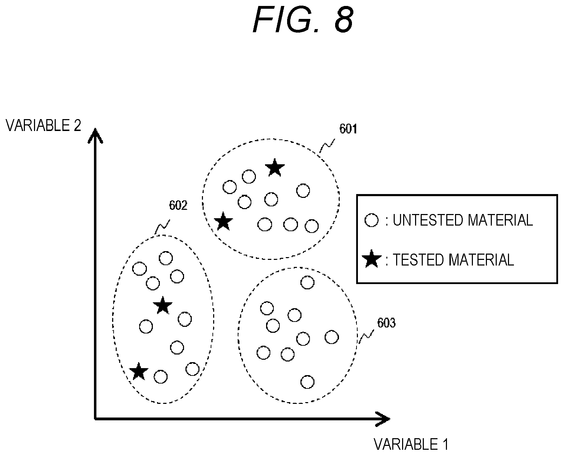

FIG. 8 schematically illustrates distribution of a material in a two-dimensional space. The circles indicate untested materials and the stars indicate tested materials. The simulation execution target selection unit 105 performs clustering of materials by similarity in the material space. Each cluster is configured of a similar material. In the example of FIG. 8, three clusters 601 to 603 are configured.

In order to satisfy the above request 1, it is preferable not to select many materials from a biased cluster but to unbiasedly select materials from different clusters. In order to satisfy the above request 2, it is preferable to preferentially select a tested material.

Therefore, the simulation execution target selection unit 105 selects a material that is a simulation execution target, for example, in accordance with the following priority order. (1) Tested material near the cluster center, (2) material in the cluster not containing any tested materials, (3) untested material near the cluster center, (4) tested material deviating from the above conditions, and (5) untested material deviating from the above conditions.

The simulation execution target selection unit 105 searches for a material that satisfies the conditions in the order of the above conditions (1) to (5), for example. The material near the cluster center is, for example, a material within a predetermined distance from the cluster center. For example, when the total number of found materials or the number of tested materials reaches a predetermined number, the simulation execution target selection unit 105 ends the search. Thus, the found material is determined as a simulation execution target and included in the material list.

Returning to FIG. 7, in step S103, the simulation execution unit 107 receives the material list from the simulation execution target selection unit 105, and executes simulation of the material in the material list to calculate the material feature value. The material list may indicate, for example, a database identifier, the number in the database, and a descriptor.

The simulation execution unit 107 acquires the chemical structural formula of the material indicated by the material list from the tested material database 102 and the untested material database 103, and executes these simulations. When a descriptor is necessary for the simulation, the simulation execution unit 107 requests the descriptor calculation unit 104 to calculate the descriptor.

In step S104, the simulation execution unit 107 stores the simulation result into the simulation result database 110. The simulation result database 110 includes, for example, a number column, a structural formula (SMILES) column, and a column of a simulation result of a material feature value. The number column identifies a record in the simulation result database 110, for example. The simulation result database 110 may indicate the presence or absence of the test result of the material.

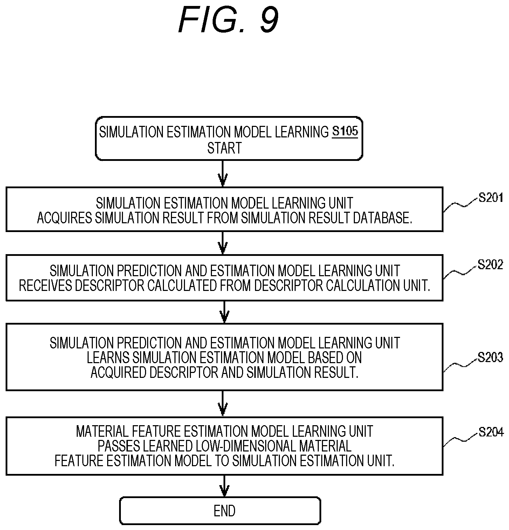

In step S105, the simulation estimation model learning unit 109 performs learning of the simulation estimation model 21 that estimates a simulation result from a descriptor. FIG. 9 illustrates a flowchart of details of learning (S105) of the simulation estimation model 21.

In step S201, the simulation estimation model learning unit 109 acquires a simulation result from the simulation result database 110. In step S202, the simulation estimation model learning unit 109 receives a calculated descriptor from the descriptor calculation unit 104. Specifically, the simulation estimation model learning unit 109 passes the chemical structural formula of the simulation to the descriptor calculation unit 104 and acquires the descriptors.

In step S203, the simulation estimation model learning unit 109 performs learning of the simulation estimation model based on the acquired descriptor and the material feature value indicated by the simulation result. The simulation estimation model learning unit 109 retains information on an initial configuration of the simulation estimation model 21 in advance, and configures the simulation estimation model in accordance with the information. A discretionary type of machine learning model can be used as the simulation estimation model 21.

The simulation estimation model learning unit 109 sequentially inputs descriptors into the simulation estimation model 21 and acquires an output simulation result estimation value (material feature value). The simulation estimation model learning unit 109 optimizes the simulation estimation model 21 by updating parameters of the simulation estimation model 21 on the basis of an error between the simulation result estimation value and the material feature value of the acquired simulation result. Finally, in step S204, the simulation estimation model learning unit 109 passes the learned simulation estimation model 21 to the simulation estimation unit 108.

Returning to FIG. 7, in step S106, the simulation estimation unit 108 receives the learned simulation estimation model 21 from the simulation estimation model learning unit 109.

The simulation estimation unit 108 further receives a descriptor of a material for which simulation has not been executed from the descriptor calculation unit 104. Specifically, the simulation estimation unit 108 selects the chemical structural formula of a material that is stored in the untested material database 103 and not stored in the simulation result database 110, and requests the descriptor calculation unit 104 to calculate the descriptor.

Furthermore, the simulation estimation unit 108 sequentially inputs the descriptors acquired from the descriptor calculation unit 104 to the learned simulation estimation model 21 to calculate an estimation value of the simulation result.

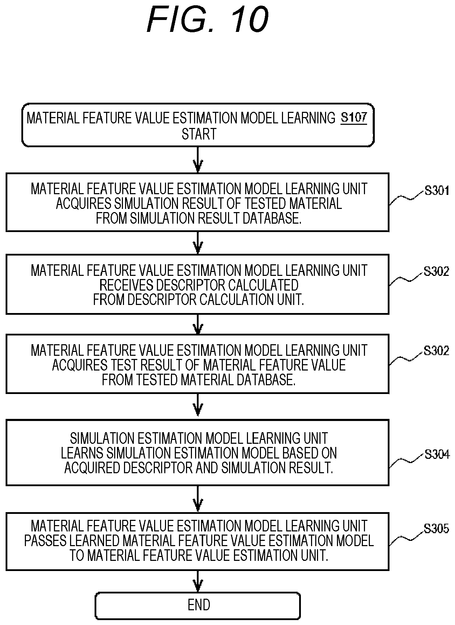

Next, in step S107, the material feature value estimation model learning unit 106 performs learning of the material feature value estimation model 25. FIG. 10 illustrates a flowchart of details of learning (S107) of the material feature value estimation model 25.

In step S301, the material feature value estimation model learning unit 106 acquires a simulation result of the tested material from the simulation result database 110. The material feature value estimation model learning unit 106 can identify a tested material by referring to the tested material database 102, for example. The simulation result database 110 may indicate the presence or absence of the test.

In step S302, the material feature value estimation model learning unit 106 receives the calculated descriptor from the descriptor calculation unit 104. Specifically, the material feature value estimation model learning unit 106 passes the chemical structural formula of the simulation result acquired in step S301 to the descriptor calculation unit 104, and acquires the descriptors.

In step S303, the material feature value estimation model learning unit 106 acquires a test result of the material feature value from the tested material database 102. Specifically, the material feature value estimation model learning unit 106 acquires, from the tested material database 102, the material feature value of the simulation result acquired in step S301.

In step S304, the material feature value estimation model learning unit 106 performs learning of the material feature value estimation model 25 based on the acquired simulation result, the acquired descriptor, and the test result of the material feature value. The simulation estimation model learning unit 109 retains information on an initial configuration of the material feature value estimation model 25 in advance, and configures the material feature value estimation model 25 in accordance with the information. A discretionary type of machine learning model can be used as the material feature value estimation model 25.

The material feature value estimation model learning unit 106 sequentially inputs, into the material feature value estimation model 25, extension descriptors (vectors) in which the descriptor and the simulation result of the material feature value are combined, and acquires the output material feature estimation value. The material feature value estimation model learning unit 106 optimizes the material feature value estimation model 25 by updating parameters of the material feature value estimation model 25 on the basis of an error between the material feature estimation value and the material feature value of the acquired test result. Finally, in step S304, the material feature value estimation model learning unit 106 passes the learned material feature value estimation model 25 to the material feature value estimation unit 111.

As described above, the learning of the material feature value estimation model 25 uses the simulation result by the numerical simulator. This makes it possible to configure the material feature value estimation model 25 that is more appropriate. In another example, the learning of the material feature value estimation model 25 may use the estimation result of the learned simulation estimation model 21.

Returning to FIG. 7, in step S108, the material feature value estimation unit 111 calculates an estimation value of the material feature value of an untested material by the learned material feature value estimation model 25. Specifically, the material feature value estimation unit 111 receives the learned material feature value estimation model 25 from the material feature value estimation model learning unit 106.

The material feature value estimation unit 111 receives a descriptor of an untested material from the descriptor calculation unit 104. For example, the material feature value estimation unit 111 acquires a chemical structural formula from the untested material database 103, and requests the descriptor calculation unit 104 to generate a descriptor together with them.

The material feature value estimation unit 111 receives the simulation result estimation value of the untested material calculated in step S106 from the simulation estimation unit 108. The material feature value estimation unit 111 acquires a simulation result of an untested material from the simulation result database 110.

The material feature value estimation unit 111 combines and inputs, to the material feature value estimation model 25, the descriptor with the simulation result estimation value (material feature value) or the simulation result (material feature value). The material feature value estimation model 25 calculates an estimation value of the feature value of the untested material represented by the input descriptor.

Finally, in step S109, the material feature estimation result display unit 112 receives the chemical structural formula of the untested material and the material feature estimation result from the material feature value estimation unit 111. The material feature estimation result display unit 112 presents the user the chemical structural formula and the material feature estimation result.

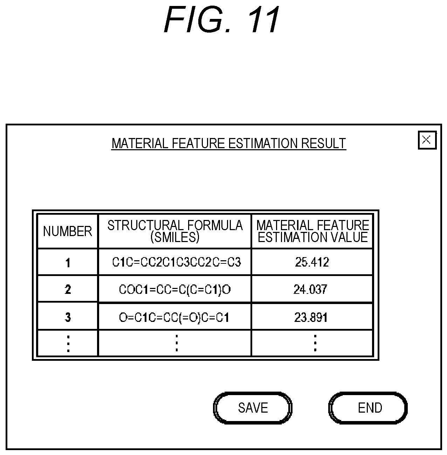

FIG. 11 illustrates an image example of the material feature estimation result displayed on the monitor 156 by the material feature estimation result display unit 112. In the example of FIG. 11, the image indicates the chemical structural formulae of the selected materials and the estimation values of the material feature values corresponding to them. With reference to the displayed chemical structural formulae and material feature values, the user can determine the chemical structural formula for actually executing a test or simulation. The estimation result is saved by a save button.

The present invention is not limited to the example described above, and includes various modifications. For example, the above-described example has been described in detail for easy understanding of the present invention, and is not necessarily limited to those having all the described configurations. A part of the configuration of a certain example can be replaced by the configuration of another example, and the configuration of another example can be added to the configuration of a certain example. A part of the configuration of each example can be added to, deleted from, or replaced by another configuration.

Some or all of the above-described configurations, functions, processing units, and the like may be implemented by hardware, for example, by designing with an integrated circuit. The above configurations, functions, and the like may be implemented by software by a processor interpreting and executing a program that implements each function. Information such as a program, a table, and a file for implementing each function can be stored in a memory, a recording device such as a hard disk and a solid state drive (SSD), or a recording medium such as an IC card and an SD card.

The control lines and the information lines indicate what is considered to be necessary for the description, and do not necessarily indicate all the control lines and the information lines on the product. In practice, almost all the configurations may be considered to be connected to one another.

## Claims

1. A system that estimates a feature value of a material, the system comprising:
one or more processors; and
one or more storage devices,
wherein the one or more storage devices store a material feature estimation model,
the material feature estimation model includes
a simulation estimation model that estimates a feature value of a simulation result of a material from a descriptor of the material, and
a material feature value estimation model that estimates a feature value of the material from an estimation result of the simulation estimation model and a descriptor of the material, and
the one or more processors
inputs a descriptor of a first material into the simulation estimation model to acquire a first simulation estimation result of a feature value of the first material, and
inputs the first simulation estimation result and a descriptor of the first material into the material feature value estimation model to acquire a feature estimation value of the first material.

2. The system according to claim 1, wherein
the one or more storage devices include
a simulator that estimates a feature value of a material by simulation, and
a tested material database that indicates a tested material associated with a measurement value of a feature value, and
the one or more processors
acquire a measurement value from the tested material database,
executes, by the simulator, simulation of a material of the acquired measurement value to acquire a simulation result, and
performs learning of the material feature estimation model by using the acquired measurement value and the simulation result.

3. The system according to claim 2, wherein
the one or more storage devices store an untested material database that indicates an untested material, and
the one or more processors select, from the tested material database and the untested material database, data to be included in learning data of the simulation estimation model and the material feature value estimation model on a basis of a similarity between materials stored in the tested material database and the untested material database.

4. The system according to claim 2, wherein
in learning of the material feature estimation model, a descriptor of a material of the acquired measurement value and the simulation result are input to the material feature value estimation model.

5. The system according to claim 1, wherein
the one or more processors output, to a monitor, information on the first material and a feature estimation value by a material feature value estimation model of the first material.

6. A method that is executed by a system, wherein
the system includes
one or more processors, and
one or more storage devices,
the one or more storage devices store a material feature estimation model,
the material feature estimation model includes
a simulation estimation model that estimates a feature value of a simulation result of a material from a descriptor of the material, and
a material feature value estimation model that estimates a feature value of the material from an estimation result of the simulation estimation model and a descriptor of the material, and
the method includes
inputting, by the one or more processors, a descriptor of a first material into the simulation estimation model to acquire a first simulation estimation result of a feature value of the first material, and
inputting, by the one or more processors, the first simulation estimation result and a descriptor of the first material into the material feature value estimation model to acquire a feature estimation value of the first material.

7. The method according to claim 6, wherein
the one or more storage devices include
a simulator that estimates a feature value of a material by simulation, and
a tested material database that indicates a tested material associated with a measurement value of a feature value, and
the method includes
acquiring, by the one or more processors, a measurement value from the tested material database,
executing, by the one or more processors, simulation of a material of the acquired measurement value by the simulator, to acquire a simulation result, and
performing, by the one or more processors, learning of the material feature estimation model by using the acquired measurement value and the simulation result.

8. The method according to claim 7, wherein
the one or more storage devices store an untested material database that indicates an untested material, and
the method includes selecting, by the one or more processors, from the tested material database and the untested material database, data to be included in learning data of the simulation estimation model and the material feature value estimation model on a basis of a similarity between materials stored in the tested material database and the untested material database.

9. The method according to claim 7, wherein
in learning of the material feature estimation model, a descriptor of a material of the acquired measurement value and the simulation result are input to the material feature value estimation model.

10. The method according to claim 6, comprising
outputting, by the one or more processors, to a monitor, information on the first material and a feature estimation value by a material feature value estimation model of the first material.
